# EUROPÄISCHE PATENTANMELDUNG

(11) **EP 0 780 161 A1**
(43) Veröffentlichungstag der Anmeldung: **25.06.1997**
(21) Anmeldenummer: 96110022.9
(22) Anmeldetag: 21.06.1996
(51) Int. Cl.: B05B 11/04, B05B 15/06, A61M 3/02, B65D 47/06

(54) **Sprühflasche insbesondere für ein Medikament**

(30) Priorität: 31.07.1995 DE 19527943
(71) Anmelder: KERPLAS NEUENBURG GMBH KUNSTSTOFFVERPACKUNGEN, D-79395 Neuenburg (DE); CCDRD - Cooperative Clinical Drug Research and Development GmbH, 16341 Zepernick (DE)
(72) Erfinder: Birmelin, Uwe, D-79424 Auggen (DE)
(74) Vertreter: Schmitt, Hans, Dipl.-Ing.

(57) **Zusammenfassung**

Eine Sprühflasche (1) mit einer elastisch nachgiebigen Wand (2), gegebenenfalls einem elastisch nachgiebigen Boden (6) hat in ihrem Inneren einen vom Boden (6) bis zur Mündung (5) reichenden Schlauch (7) und wird teilweise mit Flüssigkeit, beispielsweise einem Medikament gefüllt, so daß beim Verformen der Flasche im Sinne einer Verminderung ihres Innenvolumens durch das Innere des Schlauches (7) Flüssigkeit und an der Außenseite über Luftaustritte im Mündungsbereich Luft austreten und ein Sprühstrahl entsteht. Der die Mündung (5) aufweisende Mündungsteil (4) ist dabei gegenüber der Flasche (1) bzw. dem Flaschenhals (3) verschwenkbar und bleibt in der jeweils verschwenkten Position stehen, so daß der Sprühstrahl auch bei etwa vertikal gerichteter Flasche (1) zur Seite gelenkt wird. Dadurch eignet sich die Sprühflasche (1) zu einer Verwendung beim Einsprühen eines Medikamentes in ein Ohr durch den Benutzer selbst. Es können somit die Vorteile des Sprühens auch für Ohrtropfen nutzbar gemacht werden.

## Beschreibung

Die Erfindung betrifft eine Sprühflasche mit einem elastisch nachgiebigen Wand- und/oder Bodenbereich, mit einer im Inneren etwa vom Boden bis zu Mündung reichenden Leitung, z.B. mit einem Schlauch für den flüssigen Inhalt der Flasche, insbesondere ein Medikament, mit wenigstens einem zusätzlich zu der Leitung oder dem Schlauch an der Mündung angeordneten Luftaustritt und mit einem den Mündungsbereich bei Nichtgebrauch überdeckenden, insbesondere abdichtenden Verschluß.

Derartige Sprühflaschen sind beispielsweise zum Sprühen eines Medikamentes in die Nase bekannt. Der vom Boden bis in den Mündungsbereich reichende Schlauch macht dabei eine vertikale Halterung der Flasche während des Sprühens erforderlich, damit gleichzeitig aus der Flasche Luft und die Flüssigkeit ausgetrieben und zerstäubt werden. Dies kommt den anatomischen Verhältnissen im Nasenbereich entgegen.

In Fällen, in denen jedoch der Sprühstrahl gegenüber einer Vertikalen schräg orientiert sein müßte, versagt eine solche Flasche relativ schnell, nämlich dann, wenn während des Sprühens die untere Eintrittsöffnung in den Schlauch nicht mehr von der Flüssigkeit überdeckt ist.

Es besteht deshalb die Aufgabe, eine Sprühflasche der eingangs erwähnten Art zu schaffen, mit der ein gegenüber der Vertikalen schräger Sprühstrahl erzeugt werden kann, trotzdem aber der Flascheninhalt weitestgehend verbraucht werden kann.

Die Lösung dieser scheinbar widersprünglichen Aufgabe besteht darin, daß die Mündung an einem gegenüber der Flasche oder dem Flaschenhals aus einer mit der Achse der Sprühflasche fluchtenden Position relativ zu dieser verschwenkbaren Mündungsteil angeordnet und der Mündungsteil in verschwenkter Position haltend ist.

Auf diese Weise kann also die Richtung des Sprühstrahles relativ zur Mittelachse der Flasche schräggestellt werden, so daß bei weiterhin vertikaler Haltung der Flasche dennoch der Sprühstrahl schräg ausgebracht werden kann.

Besonders günstig ist es dabei, wenn unterschiedliche Schwenkwinkel und/oder unterschiedliche Schwenkstellungen des Mündungsteiles einstellbar sind. Dadurch kann die Richtung des Sprühstrahles je nach Bedarf verändert werden.

Es ergibt sich also auf vorteilhafte Weise eine Sprühflasche mit schwenkbarem Mündungsteil, die zum Einsprühen eines Medikamentes durch den Benutzer selbst in sein Ohr verwendet werden kann.

Bisher müssen Ohrentropfen bei unbequemer Kopfhaltung in das Ohr geträufelt werden, was zu einer Überdosierung des Medikamentes führt, da zur Benetzung des Innenraumes des Ohres gewissermaßen der entsprechende Raum mit der Flüssigkeit mehr oder weniger gefüllt werden muß. Die erfindungsgemäße Sprühflasche erlaubt nun ein Einsprühen in das Ohr des Benutzers durch diesen selbst, ohne daß er den Kopf neigen oder stark neigen muß. Dabei kann er die Mündung auf seine anatomischen Gegebenheiten so einstellen, daß er ganz bequem den Sprühstrahl in sein Ohr richten und einsprühen kann, wobei in vorteilhafter Weise durch das Versprühen das Medikament das Ohr im Inneren gut benetzt, ohne es ausfüllen zu müssen.

Besonders zweckmäßig ist es dabei, wenn der Abstand zwischen der Mündung und der Schwenkachse größer als die horizontale Abmessung zwischen dieser Schwenkstelle und der äußeren Wand der Flasche ist, so daß zumindest nach einer gewissen Verschwenkung des Mündungsteiles die Mündung die Flaschenaußenseite überragt. Dies ermöglicht es, diese Mündung von der Seite her etwas in das Ohr einzuführen, gleichzeitig die Flasche selbst aber für eine bestmögliche Versprühung vertikal zu halten.

Die Verschwenkbarkeit des Mündungsteiles relativ zu der Sprühflasche bzw. dem eigentlichen Flaschenkörper kann auf verschiedene Weisen realisiert sein. Beispielsweise kann eine knickbare gewellte Membran oder ein Faltenbalg den Mündungsteil gegenüber dem Flaschenkörper schwenkbar halten. Somit wird der Mündungsteil über eine Membran oder einen Faltenbalg zwar im wesentlichen vertikal gehalten, kann aber aufgrund der gewellten oder knickbaren Membran bzw. aufgrund des Faltenbalges aus seiner axialen Position zur Seite verschwenkt werden, wie man es beispielsweise von Trinkröhrchen kennt.

Dabei kann die Membrane oder der Faltenbalg nach einer Seite einknickbar und auf der am Mündungsteil gegenüberliegenden Seite streckbar sein, so daß sich dadurch eine Festlegung der Schwenkposition ergibt. Vor allem die Dichtigkeit im Bereich des Schwenkgelenkes kann auf diese Weise besonders gut sein, weil eine geschlossene Wandung auch am Übergang von der Flasche zu dem Mündungsteil vorhanden ist.

Besonders vorteilhaft ist es jedoch, wenn der Mündungsteil mit dem Flaschenkörper über ein Walzen- oder Kugelgelenk verbunden ist. Dies läßt sich gut abdichten und erlaubt stufenlose beliebige Verschwenkwinkel des Mündungsteiles und im Falle eines Kugelgelenkes auch nach beliebigen Seiten hin, was vor allem bei Flaschen mit unrundem Querschnitt vorteilhaft sein kann. Man kann dann bevorzugt nach der Seite der Flasche schwenken, die die geringere Seitenabmessung hat.

Dabei kann die Kugel oder Walze bevorzugt an der Flasche selbst vorgesehen sein und das Mündungsteil kann die sie aufnehmende Pfanne aufweisen. Dies hat den Vorteil, daß der Druck des Verschlusses auf den Mündungsteil in seiner Gebrauchsstellung von dem Gelenk gut aufgenommen werden kann.

Es ist aber auch denkbar, eine Pfanne oder Lagerung in einen Flaschenhals oder dergleichen einzuarbeiten und den Mündungsteil mit der Kugel oder Walze zu versehen, die in dieser Pfanne gelagert ist. Dies erlaubt eine einfachere Herstellung vor allem der Flasche, weil am Flaschenhals lediglich innenseitig Einbuchtungen zur Aufnahme einer kugeligen oder walzenförmigen Verdickung des Mündungsteiles vorgesehen werden müssen, wobei auch der Mündungsteil relativ einfach herstellbar ist, indem an seinem der Mündung abgewandten Ende die kugelige oder walzenförmige Anformung vorzusehen ist, die im Inneren für den Durchlaß des Schlauches und der Luft hohl ist.

Die an der Flasche befindliche Kugel oder Walze kann entweder an einem in dem Hals der Flasche befindlichen, über den Flaschenhals hinausragenden Einsatz einstückig befestigt sein oder sie kann an einem am Flaschenhals anschraubbaren Ansatz vorgesehen sein, der seinerseits ein Außengewinde für die Verschlußkappe trägt. Im ersteren Falle übergreift dann der Verschluß den Mündungsteil und die Außenseite des Flaschenhalses, wo er verschraubt werden kann. Im zweiten Falle läßt sich das die Kugel oder Walze tragende Teil an den Flaschenhals verschrauben und trägt dann seinerseits eine kleinere Verschlußkappe. Vor allem für ein Nachfüllen ist eine solche Lösung, die das Abschrauben des die Kugel aufweisenden Teiles erlaubt, günstiger.

Das den Schlauch und die Luftöffnung enthaltende Mündungsteil ist in an sich bekannter Weise etwas konisch und mit einem runden Querschnitt versehen, um gut an das Ohr angepaßt zu sein.

Die Schraubkappe kann in an sich bekannter Weise einen Innenkonus haben, der den konischen Mündungsteil in Gebrauchsstellung überdeckt und abdichtet. Dies hat den Vorteil, daß der Benutzer die zunächst verschwenkte Mündung beim Verschließen der Flasche nicht zuerst wieder geradestellen muß oder mindestens nicht genau in die gerade Position bringen muß, sondern daß diese Rückverschwenkung durch das Aufsetzen und Aufschrauben der Schraubkappe automatisch erfolgt.

Insgesamt ergibt sich dabei eine Sprühflasche, die in bekannter Weise von oben her bequem befüllt und maschinell verschlossen werden kann, trotzdem aber vom Benutzer dann individuell auf seine anatomischen Gegebenheiten z.B. am Ohr eingestellt werden kann.

Nachstehend sind mehrere Ausführungsbeispiele der Erfindung mit ihren ihr als wesentlichen zugehörenden Einzelheiten anhand der Zeichnung näher beschrieben. Es zeigt in zum Teil schematisierter Darstellung:
- Fig.1: eine Sprühflasche mit abgenommener Verschlußkappe und gegenüber einer ursprünglich vertikalen Position seitwärts verschwenktem Mündungsteil oder Mündungsstück im Längsschnitt,
- Fig.2: einen gegenüber Fig.1 um 90° versetzten Längsschnitt einer erfindungsgemäßen Sprühflasche in verschlossenem Zustand, wobei eine Kugel eines Kugelgelenkes für den verschwenkbaren Mündungsteil an einem am Hals der Flasche anschraubbaren Ansatz angeordnet ist,
- Fig.3: eine Draufsicht der Sprühflasche gemäß Fig.2,
- Fig.4: einen Längsschnitt einer geöffneten Sprühflasche mit verschwenktem Mündungsteil, wobei in den Flaschenhals ein Einsatz eingefügt ist, der einstückig die Kugel für das Kugelgelenk trägt,
- Fig.5: einen um 90° gedrehten Längsschnitt der Sprühflasche gemäß Fig.1, jedoch in Verschlußposition, bei welcher die Verschlußkappe den senkrecht gestellten, mit der Flaschenachse fluchtenden Mündungsteil übergreift und abdichtet,
- Fig.6: einen Längsschnitt einer Sprühflasche mit zugehöriger, abgenommener Verschlußkappe und verschwenktem Mündungsteil, wobei der Mündungsteil die Kugel des Kugelgelenkes aufweist, die in einer entsprechenden Einformung des Flaschenhalses als Kugelpfanne gelagert ist,
- Fig.7: einen um 90° gegenüber Fig.6 verdreht angeordneten Längsschnitt der Sprühflasche, wobei diese verschlossen ist und die Verschlußkappe den zurückverschwenkten Mündungsteil übergreift und an der Mündung abdichtet,
- Fig.8: einen Längsschnitt einer abgewandelten Sprühflasche, bei welcher der Mündungsteil mit einem an dem Flaschenhals angeschraubten Ansatz einstückig über eine knickbare Membran verbunden ist, die in geknickter, den Mündungsteil verschwenkt haltender Position dargestellt ist, wobei wiederum die Schraubkappe abgenommen ist, sowie
- Fig.9: einen um 90° verdrehten Längsschnitt der Sprühflasche gemäß Fig.8 bei in Flucht mit der Flaschenachse geschwenktem Mündungsteil, wobei die Verschlußkappe abdichtend aufgeschraubt ist.

Bei den nachfolgend beschriebenen Ausführungsbeispielen sind in ihrer Funktion übereinstimmende Teile auch bei abweichender Gestaltung mit übereinstimmenden Bezugszahlen versehen.

Eine im ganzen mit 1 bezeichnete Sprühflasche wird im folgenden auch kurz "Flasche 1" genannt. Sie hat einen elastisch nachgiebigen Wandbereich 2, einen Flaschenhals oder Hals 3 und an einem Mündungsteil 4 eine Mündung 5. Von ihrem Boden 6 verläuft im Inneren der Flasche 1 eine Leitung oder ein Schlauch 7 zu der Mündung 5. Darüber hinaus ist in nicht näher dargestellter Weise an der Mündung 5 ein Luftaustritt, so daß an der Mündung 5 beim Verformen des Wandbereiches 2 mittels Druck an der Mündung 5 teilweise Flüssigkeit durch den Schlauch 7, teilweise Luft durch den Luftaustritt austritt und die gewünschte Zerstäubung der Flüssigkeit bewirkt. Bei Nichtgebrauch ist die Mündung 5 und der gesamte Mündungsteil 4 von einem als Kappe ausgebildeten Verschluß 8 dichtend überdeckt, wie man es in den Figuren 2, 5, 7 und 9 erkennt. Der Verschluß 8 ist dabei in all diesen Ausführungsbeispielen als Gewindekappe mit Innengewinde 9 ausgebildet, das zu einem Außengewinde 10 an der Flasche 1 paßt. Somit kann in verschraubter Position von dem Verschluß 8 ein gewisser Druck auf die Mündung 5 ausgeübt werden, also eine gute Abdichtung erzielt werden.

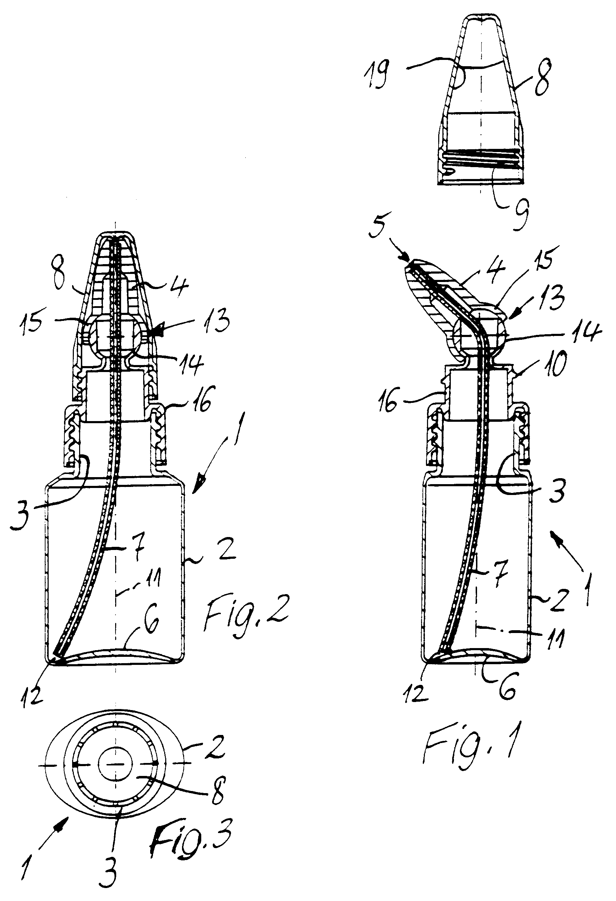

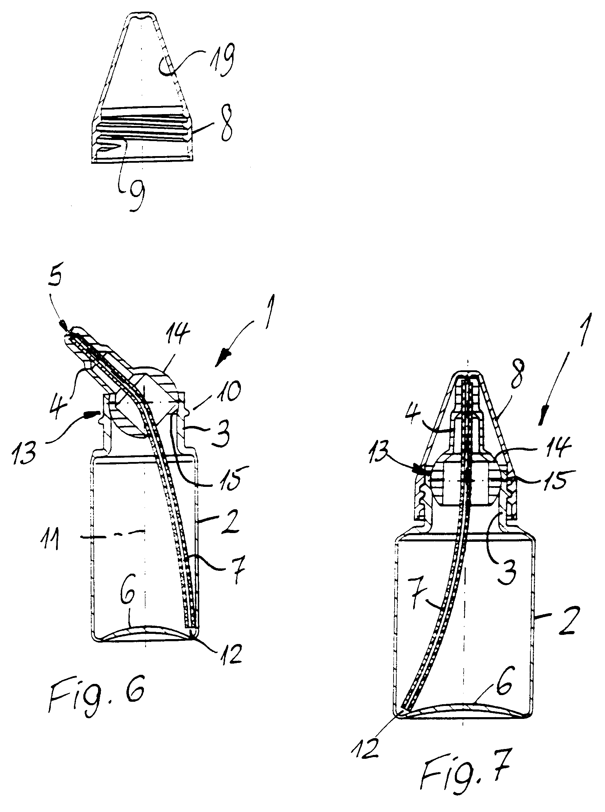

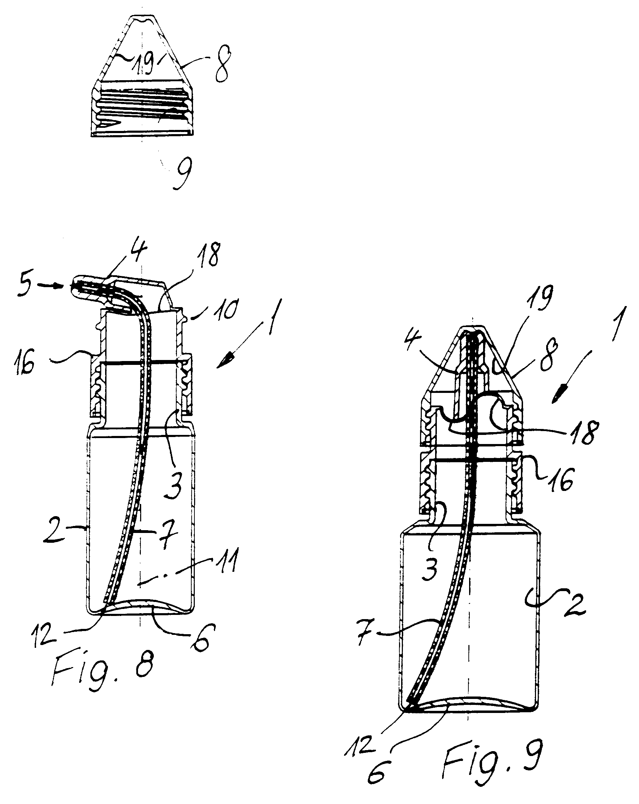

In allen Ausführungsbeispielen ist vorgesehen, daß die Mündung 5 an einem gegenüber der Flasche 1 in unterschiedlicher Weise verschwenkbaren Mündungsteil 4 angeordnet und der Mündungsteil 4 auch in verschwenkter Position haltend ist, sei es durch Reibkräfte innerhalb eines noch zu beschreibenden Gelenkes bei den Ausführungsbeispielen gemäß Fig.1 bis 7, sei es durch eine Selbsthaltung eines einknickbaren Bereiches beim Ausführungsbeispiel gemäß Fig.8 und 9.

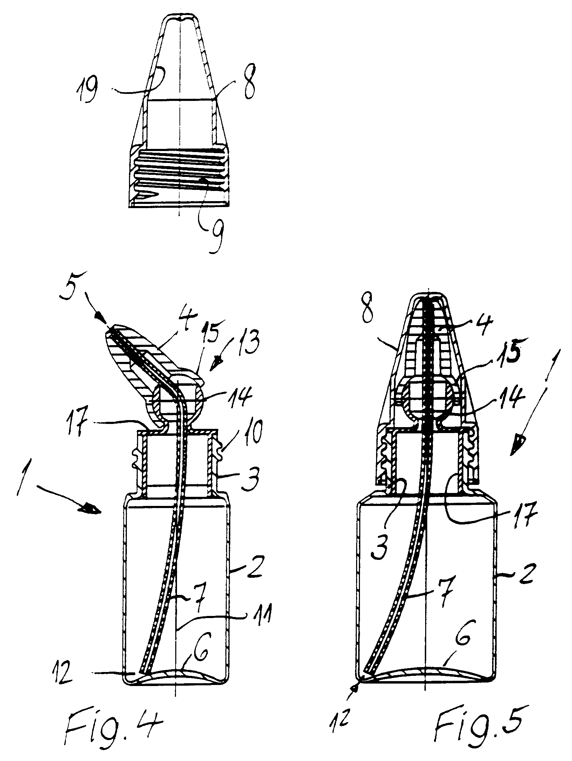

Der an der Mündung 5 austretende Sprühstrahl kann also relativ zur Mittelachse 11 der Flasche 1, die gemäß Fig.3 nicht unbedingt einen kreisrunden Querschnitt haben muß, sondern auch oval oder in sonstiger Weise konturiert sein kann, schräg gerichtet werden. Somit kann der Benutzer die Flasche 1 etwa senkrecht oder vertikal halten und dennoch den Sprühstrahl nach der Seite richten. Dies ist besonders günstig, wenn der Benutzer mit der Flasche 1 ein Medikament in sein Ohr einsprühen möchte. Er kann die Vorteile des Einsprühens also auch bei einer anatomischen Öffnung zur Anwendung bringen, die nicht von unten nach oben gerichtet ist wie beispielsweise Nasenlöcher. Dennoch kann das Prinzip der Sprühflasche ausgenutzt werden, bei welcher es vor allem gegen Ende der Entleerung wichtig ist, daß der Flüssigkeitsspiegel oberhalb des unteren Eintrittes 12 des Schlauches 7 bleibt, damit die Flasche 1 möglichst vollständig entleert werden kann.

Es ist gut erkennbar, daß bei den Ausführungsformen gemäß den Figuren 1 bis 7 unterschiedliche Schwenkwinkel und auch unterschiedliche Schwenkstellungen des Mündungsteiles 4 einstellbar sind. In Fig.1, 4 und 6 sind dabei jeweils die größtmöglichen Schwenkstellungen dargestellt, während die Figuren 2, 5 und 7 die andere Endstellung, also die unverschwenkte Stellung zeigt. Selbstverständlich sind dazwischen auch beliebige Zwischenpositionen möglich, da die noch zu beschreibenden Gelenke 13 eine stufenlose Verschwenkung jeweils nach beliebigen Seiten erlauben. Der Benutzer kann also die Richtung des Sprühstrahles je nach Bedarf zwischen den jeweils in Fig.1 und 2 oder Fig.4 und 5 oder Fig.6 und 7 dargestellten Endstellungen und selbstverständlich auch in diesen Endstellungen selbst wählen und auf seine anatomischen Bedürfnisse und Anwendungsfälle anpassen.

Dabei verdeutlichen die Figuren 1, 4, 6 und 8, daß der Abstand zwischen der Mündung 5 und der Schwenkachse des Kugelgelenkes 13 oder einer andersartigen Verschwenkmöglichkeit (Fig.8 und 9) und die Länge des Mündungsteiles 4 größer als die horizontale Abmessung zwischen der Schwenkstelle und der äußeren Wand 2 der Flasche 1 zumindest in Richtung der kleineren Flaschenabmessung ist. Mit anderen Worten, zumindest nach einer gewissen Verschwenkung des Mündungsteiles 4 überragt dieses die Wand 2 der Flasche 1, das heißt die Mündung 5 hat in senkrechter Projektion gegenüber der Flaschenwand 2 einen Überstand, so daß ein Benutzer die Mündung 5 nahe oder teilweise sogar in die Öffnung seines Ohres einführen kann, ohne daß dies durch die Flasche 1 verhindert wird. Entsprechend effektiv kann ein Sprühstrahl von dem Benutzer in seinem Ohr appliziert werden.

In den Ausführungsbeispielen gemäß Fig.1 bis 7 ist der Mündungsteil 5 mit dem Flaschenkörper bzw. dem Flaschenhals 3 über das schon erwähnte Kugelgelenk 13 verbunden. Dies läßt sich gut abdichten und erlaubt die schon erwähnte stufenlose Verschwenkung des Mündungsteiles 4 nach beliebigen Seiten und innerhalb der Endstellungen um beliebige Winkel. Vor allem bei Flaschen 1 mit unrundem Querschnitt gemäß Fig.3 ist dies vorteilhaft, weil sich so das Mündungsteil 4 nach der Seite der Flasche 1 verschwenken läßt, die die geringere Seitenabmessung hat, damit auch schon nach einem kleineren Schwenkwinkel die Mündung 5 außerhalb des eigentlichen Flaschenumrisses zu liegen kommt und in die Öffnung eines Ohres eingeführt werden kann, ohne die Flasche 1 selbst schrägstellen zu müssen.

Da der Schlauch 7 jedoch mit seiner Eintrittsöffnung 12 unter Umständen im Randbereich des Bodens 6 angeordnet ist, kann sogar eine gewisse Neigung der Flasche bei ihrer letzten Entleerung günstig sein, wenn nämlich die Flasche so geneigt wird, daß der Eintrittsbereich des Schlauches 7 dadurch gegenüber der in den Zeichnungen dargestellten schrägen Position etwa vertikal gestellt wird. Nach derselben Seite, nach der der Schlauch 7 in seinem Eintrittsbereich seitlich von der Mittelachse 11 der Flasche abweicht, kann auch der Mündungsteil 4 verschwenkt werden, so daß sich die leichte Schrägstellung der Flasche 1 und die Verschwenkung des Mündungsteiles 4 addieren können. Dies kann zu einem nahezu horizontalen Sprühstrahl an der Mündung 5 führen, falls es die Anatomie eines Ohres oder einer sonstigen Eintrittsöffnung, beispielsweise auch des Mundes für ein Mundwasser oder dergleichen zweckmäßig und vorteilhaft erscheinen läßt. Dabei ist vorteilhaft, wenn die Flasche 1 zumindest im Bereich des Eintrittes 12 durchsichtig ist.

In den Ausführungsbeispielen gemäß Fig.1 bis 5 ist die Kugel 14 des Kugelgelenkes 13 an der Flasche 1 selbst vorgesehen und das Mündungsteil 4 hat die dazu passende, die Kugel 14 übergreifende Kugelpfanne 15. Selbst bei einem Druck von oben durch die Verschlußkappe 8 ergibt dies einen stabilen Sitz, da sich der Mündungsteil 4 auch auf der Kugel 14 und mit der Außenseite seiner Pfanne 15 an der Innenseite der Kappe 8 abstützen kann.

In Fig.1 und 2 ist eine Lösung dargestellt, bei welcher die an der Flasche 1 befindliche Kugel 14 an einem am Flaschenhals 3 anschraubbaren Ansatz 16 vorgesehen ist, der seinerseits das Außengewinde 10 für die Verschlußkappe 8 trägt. Diese Anordnung hat den Vorteil, daß man auch den Ansatz 16 mit der Kugel 14 von der Flasche 1 lösen kann, um diese wieder nachfüllen zu können. Darüber hinaus ist auch bei einer nur einmal zu verwendenden Flasche 1 diese Anordnung für das Füllen und anschließende Verschließen günstig.

Im Ausführungsbeispiel gemäß Fig.4 und 5 ist vorgesehen, daß die an der Flasche 1 befindliche Kugel 14 an einem in dem Hals 3 der Flasche 1 befindlichen, über den Flaschenhals 3 hinausragenden Einsatz 17 einstückig befestigt ist. Ein Nachfüllen ist dabei nur möglich, wenn der Einsatz 17 aus dem Flaschenhals 3 herausgezogen werden kann, jedoch ist das erste Verschließen der Flasche 1 nach ihrer ersten Füllung besonders einfach, da der Einsatz 17 nur von oben her in den Flaschenhals 3 eingedrückt oder eingepreßt werden muß, was auch zu einer guten Abdichtung führt.

In Fig.8 und 9 ist ein Ausführungsbeispiel dargestellt, bei welchem eine knickbare gewellte Membran 18 den Mündungsteil 4 gegenüber dem Flaschenkörper schwenkbar hält, wobei diese Membran 18 einstückig mit einem am Flaschenhals 3 verschraubbaren Ansatz 16 verbunden ist. Es könnte aber auch in diesem Falle ein der Anordnung gemäß Fig.4 und 5 entsprechender Einsatz 17 mit einer solchen Membran 18 vorgesehen sein. Der Ansatz 16 hat dabei wiederum oberhalb seiner eigenen Verschraubung mit dem Flaschenhals ein Außengewinde 10 für das Innengewinde 9 der Schraubkappe 8. Diese knickbare Membran kann dabei eine bevorzugte Knick- und Halterichtung haben, bei der der Mündungsteil 4 in der in Fig.8 erkennbaren Weise nach derselben Seite verschwenkt wird, nach der auch die Eintrittsöffnung 12 des Schlauches 7 von der Mitte 11 der Flasche 1 abweicht. Dies ist dabei auch diejenige Seite der Flasche 1, die die geringere Seitenabmessung hat. Somit können auch bei dieser Anordnung die schon beschriebenen Vorteile beim Einsprühen eines Medikamentes in ein Ohr erreicht werden.

Das den Schlauch 7 und die Luftaustrittsöffnungen enthaltende Mündungsteil 4 ist in allen Ausführungsbeispielen in an sich bekannter Weise etwas konisch zu der Mündung 5 zulaufend und mit einem runden Querschnitt versehen, so daß es sich gut an das Ohr des Benutzers anpaßt und teilweise darin einführen läßt. Die Verschlußkappe 8 hat jeweils einen Innenkonus 19, der den konischen Mündungsteil 4 in Gebrauchsstellung überdeckt und abdichtet und dabei gemäß Fig.2, 5, 7 und 9 auch außenseitig berührt und zentriert. Der Benutzer braucht somit den Mündungsteil 5 nach der Benutzung nicht wieder in die mit der Mittelachse 11 fluchtende Position zurückzuschwenken, sondern er braucht nur eine Teilverschwenkung oder sogar gar keine Verschwenkung des Mündungsteiles 5 durchzuführen, weil er die Verschlußkappe 8 über den noch schrägen Mündungsteil 4 stülpen und dann so verschwenken kann, daß das Innengewinde 9 das Außengewinde 10 erfaßt. Beim weiteren Verschrauben wird dann auch der Mündungsteil 4 automatisch in die ausgerichtete Position verschwenkt und zentriert. Das Rückverschwenken des Mündungsteiles 5 kann also durch das Aufsetzen und Aufschrauben der Verschlußkappe 8 automatisch erfolgen.

Statt der hohlen Kugel 14 könnte das Gelenk 13 auch eine hohle Walze aufweisen, deren Querschnitt eine den Fig.1, 4 und 6 entsprechende Form haben könnte, während die Querschnitte rechtwinklig dazu unrund wären. Dies würde aber nur Verschwenkungen in zwei einander entgegengesetzte Richtungen erlauben.

Die Sprühflasche 1 mit einer elastisch nachgiebigen Wand 2, gegebenenfalls einem elastisch nachgiebigen Boden 6 hat in ihrem Inneren einen vom Boden 6 bis zur Mündung 5 reichenden Schlauch 7 und wird teilweise mit Flüssigkeit, beispielsweise einem Medikament gefüllt, so daß beim Verformen der Flasche im Sinne einer Verminderung ihres Innenvolumens durch das Innere des Schlauches 7 Flüssigkeit und an der Außenseite über Luftaustritte im Mündungsbereich Luft austreten und ein Sprühstrahl entsteht. Der die Mündung 5 aufweisende Mündungsteil 4 ist dabei gegenüber der Flasche 1 bzw. dem Flaschenhals 3 verschwenkbar und bleibt in der jeweils verschwenkten Position stehen, so daß der Sprühstrahl auch bei etwa vertikal gerichteter Flasche 1 zur Seite gelenkt wird. Dadurch eignet sich die Sprühflasche 1 zu einer Verwendung beim Einsprühen eines Medikamentes in ein Ohr durch den Benutzer selbst. Es können somit die Vorteile des Sprühens auch für Ohrtropfen nutzbar gemacht werden.

## Patentansprüche

1. Sprühflasche (1) mit einem elastisch nachgiebigen Wand- und/oder Bodenbereich, mit einer im Inneren etwa vom Boden (6) bis zur Mündung (5) reichenden Leitung, zum Beispiel mit einem Schlauch (7) für den flüssigen Inhalt der Flasche (1), insbesondere ein Medikament, mit wenigstens einem zusätzlich zu der Leitung oder dem Schlauch (7) an der Mündung (5) angeordneten Luftaustritt und mit einem den Mündungsbereich bei Nichtgebrauch überdeckenden, insbesondere abdichtenden und lösbaren Verschluß (8), **dadurch gekennzeichnet,** daß die Mündung (5) an einem gegenüber der Flasche (1) oder dem Flaschenhals (3) aus einer mit der Achse (11) der Sprühflasche (1) fluchtenden Position relativ zu dieser verschwenkbaren Mündungsteil (4) angeordnet und der Mündungsteil (4) in verschwenkter Position haltend ist.

2. Sprühflasche nach Anspruch 1, dadurch gekennzeichnet, daß unterschiedliche Schwenkwinkel und/oder unterschiedliche Schwenkstellungen des Mündungsteiles (4) einstellbar sind.

3. Sprühflasche nach Anspruch 1 oder 2, dadurch gekennzeichnet, daß der Abstand zwischen der Mündung (5) und der Schwenkachse größer als die horizontale Abmessung zwischen dieser Schwenkstelle und der äußeren Wand (2) der Flasche (1) ist.

4. Sprühflasche nach einem der Ansprüche 1 bis 3, dadurch gekennzeichnet, daß eine knickbare, gewellte Membran (18) oder ein Faltenbalg den Mündungsteil (4) gegenüber dem Flaschenkörper oder Flaschenhals (3) schwenkbar hält.

5. Sprühflasche nach Anspruch 4, dadurch gekennzeichnet, daß die Membran (18) oder der Faltenbalg nach einer Seite einknickbar und auf der am Mündungsteil (4) gegenüberliegenden Seite streckbar ist.

6. Sprühflasche nach einem der Ansprüche 1 bis 3, dadurch gekennzeichnet, daß der Mündungsteil (4) mit der Flasche (1) oder dem Flaschenhals (3) über ein Gelenk (13), insbesondere ein Kugel- oder Walzengelenk, verbunden ist.

7. Sprühflasche nach einem der Ansprüche 1 bis 3 oder 6, dadurch gekennzeichnet, daß die Kugel (14) des oder Walze des Gelenkes (13) an der Flasche (1) selbst angeordnet oder befestigt ist und das Mündungsteil (4) die dazu passende Kugelpfanne (15) oder Lagerung aufweist.

8. Sprühflasche nach einem der Ansprüche 1 bis 3 oder 6, dadurch gekennzeichnet, daß die Kugelpfanne (15) oder Lagerung des Gelenkes (13) an dem Flaschenhals (3) eingearbeitet und der Mündungsteil (4) mit der dazu passenden Kugel (14) versehen ist.

9. Sprühflasche nach einem der Ansprüche 1 bis 8, dadurch gekennzeichnet, daß die Kugel (14) oder Walze an einem in den Hals (3) der Flasche (1) befindlichen, über den Flaschenhals (3) hinausragenden Einsatz (17) oder an einem an dem Flaschenhals (3) anschraubbaren Ansatz (16) insbesondere einstückig befestigt ist.

10. Sprühflasche nach Anspruch 9, dadurch gekennzeichnet, daß das Außengewinde (10) für die Verschlußkappe (8) an dem die Kugel (14) oder Walze tragenden Ansatz (16) vorgesehen ist.

11. Sprühflasche nach einem der Ansprüche 1 bis 10, dadurch gekennzeichnet, daß das den Schlauch (7) und die Luftaustrittsöffnung aufweisende Mündungsteil (4) zu der Mündung (5) hin konisch und mit einem runden Querschnitt versehen ist.

12. Sprühflasche nach einem der Ansprüche 1 bis 11, dadurch gekennzeichnet, daß die Verschlußkappe (8) einen Innenkonus (19) hat, der den konischen Mündungsteil (4) in Gebrauchsstellung überdeckt, abdichtet und insbesondere zentriert.

13. Sprühflasche nach einem der Ansprüche 1 bis 12, dadurch gekennzeichnet, daß sie zumindest im Bereich des Eintrittes (12) in den Schlauch (7) oder dergleichen durchsichtig ist.

14. Verwendung einer Sprühflasche mit schwenkbarem Mündungsteil (4) zum Einsprühen eines Medikamentes durch den Benutzer in sein Ohr.
